# EUROPEAN PATENT APPLICATION

(11) **EP 3 669 758 A1**
(43) Date of publication of application: **24.06.2020**
(21) Application number: 18213475.9
(22) Date of filing: 18.12.2018
(51) Int. Cl.: A61B 5/00, A61B 8/08, A61B 8/00, A61B 5/0402, A61B 5/06

(54) **ELECTROCARDIOGRAPHIC IMAGING SYSTEM**

(71) Applicant: Koninklijke Philips N.V., 5656 AG Eindhoven (NL)
(72) Inventor: CLUITMANS, Matthijs, 5656 AE Eindhoven (NL); GIJSBERS, Gerardus Henricus Maria, 5656 AE Eindhoven (NL)
(74) Representative: de Haan, Poul Erik

(57) **Abstract**

The invention relates to an electrocardiographic imaging system (100) for providing electrocardiographic image data indicative of an electrical activity map of a heart (101) suitable for real time applications, and that comprises an ultrasound imaging unit (102) for generating a three dimensional cardiac anatomical model, an electrocardiographic sensing unit (108) comprising a set of between three and twenty electrocardiographic electrodes (110) for providing body surface signals indicative of an electrical activity of the heart, a camera unit (114) for determining positions of the electrocardiographic electrodes , a transducer position determination unit (116), for determining a position of the ultrasound transducer, a torso-heart geometry determination unit (118) for determining a position and orientation of the three dimensional shape of the heart within a predetermined three dimensional coordinate space and an electrical activity determination unit (120) for generating an electrical activity map of the heart, suitable for real time applications.

## Description

### FIELD OF THE INVENTION

The invention is directed to an electrocardiographic imaging apparatus, to an electrocardiographic imaging system, to a method for controlling an electrocardiographic imaging system and to a computer program.

### BACKGROUND OF THE INVENTION

Cardiac arrhythmias are groups of conditions where the heartbeat of a living being, such as a human, is irregular, too slow or too fast. Diagnosis of some arrhythmias by a healthcare professional, such as ventricular tachycardia (VT) and premature ventricular complexes (PVCs), can be supported with information and/or data received from invasive catheter procedures. Invasive related catheter procedure generally require that for every data collection during an arrhythmia, a catheter needs to be positioned in a stable way during a full beat before it is moved to a new position where it will, again, need to remain stable for a full beat; data collection is thus point-by-point and beat-by-beat.

Electrocardiographic imaging is a noninvasive imaging modality for mapping the electric activity of the heart in humans based on a reconstruction of epicardial potentials, electrograms, and isochrones from electrocardiographic body-surface potentials noninvasively.

The article "Rapid construction of a patient-specific torso model from 3D ultrasound for non-invasive imaging of cardiac electrophysiology" L.K. Cheng et al. Med. Biol. Eng. Comput., 2005, 43, 325-330, MBEC online number: 20054000 describes the use of inverse methods for obtaining a patient-specific geometric model of the heart and torso using a pseudo-3D ultrasound system and a hand-held laser scanner for locating 256 electrocardiographic electrodes and for reconstructing the geometric model in under 20 minutes. The geometric model can then be used non-invasively to reconstruct an electrical activity map of the heart.

It is a drawback of known mapping systems and methods that the information gathered by or derived from current means is not local nor precise enough to optimally support the navigation and mapping of invasive catheters, for instance during an arrhythmia related invasive catheter procedure.

### SUMMARY OF THE INVENTION

It would be beneficial to provide a system for generating a three dimensional electrical activity map of the heart that is suitable for real-time visualization applications.

According to a first aspect of the present invention, an electrocardiographic imaging apparatus is described. The electrocardiographic imaging apparatus comprises a plurality of input units configured to receive data, such as (i) an ultrasound input unit for receiving a three dimensional cardiac anatomical model representing a three dimensional shape of the heart of the living being based on ultrasound image data, (ii) a camera input unit for receiving a respective position of each of the electrocardiographic electrodes within a predetermined three dimensional coordinate space based on image data, and (iii) a transducer input unit for receiving a position of the ultrasound transducer within the predetermined three dimensional coordinate space.

Additionally, the electrocardiographic imaging apparatus comprises an echocardiographic sensing unit. This echocardiographic sensing unit comprises a set of between three and twenty electrocardiographic electrodes for providing electrical signals; and an electrocardiographic processing unit configured to provide, when the set of electrocardiographic electrodes is positioned on a body surface of the living being, a set of body surface signals indicative of an electrical activity of the heart.

The electrocardiographic imaging apparatus further comprises a torso-heart geometry determination unit which is configured to determine, based on (i) the determined position of the ultrasound transducer, (ii) the determined positions of the electrocardiographic electrodes and (iii) the three dimensional cardiac anatomical model, a position and orientation of the three dimensional shape of the heart within the predetermined three dimensional coordinate space.

The electrocardiographic imaging apparatus further comprises an electrical activity determination unit which is configured (i) to receive the position and orientation of the three dimensional shape of the heart within the predetermined three dimensional coordinate space and the set of body surface signals; (ii) to generate an electrical activity map by using the body surface signals and the three dimensional cardiac anatomical model; and (iii) provide electrocardiographic image data indicative thereof.

The electrocardiographic imaging apparatus herein described is thus advantageously suited to provide, within a relatively simple arrangement, a real-time global cardiac electrical activity map in the three dimensional coordinate space which requires much computational effort to obtain, than in the case of typical electrocardiographic systems that require the use of 60-256 body surface electrodes.

According to a second aspect of the present invention, an electrocardiographic imaging system is described. The electrocardiographic imaging system comprises an ultrasound imaging unit that includes an ultrasound transducer configured to provide ultrasound image data of a heart of a living being and an ultrasound image data processing unit configured to generate, based on the ultrasound image data, a three dimensional cardiac anatomical model representing a three dimensional shape of the heart of the living being.

The electrocardiographic imaging system also comprises an electrocardiographic sensing unit that includes a set of between three and twenty electrocardiographic electrodes for providing electrical signals, and an electrocardiographic processing unit configured to provide, when the set of electrocardiographic electrodes is positioned on respective locations on a body surface of the living being, a set of body surface signals indicative of an electrical activity of the heart.

Additionally, the electrocardiographic imaging system comprises a camera unit, which is configured to generate image data and to determine, based on the generated image data, a respective position of each of the electrocardiographic electrodes within a predetermined three-dimensional coordinate space.

Further, the electrocardiographic imaging system comprises a transducer position determination system, which is configured to determine a position of the ultrasound transducer within the predetermined three-dimensional coordinate space.

The electrocardiographic imaging system also comprises a torso-heart geometry determination unit which is configured to determine, based on the determined position of the ultrasound system, on the determined positions of the electrocardiographic electrodes and on the three dimensional cardiac anatomical model, a position and orientation of the three dimensional shape of the heart within the predetermined three dimensional coordinate space.

Additionally, the electrocardiographic imaging system comprises an electrical activity determination unit which is configured to receive the position and orientation of the three dimensional shape of the heart within the predetermined three dimensional coordinate space and also the set of body surface signals indicative of the electrical activity of the heart The electrical activity determination unit is further configured to generate an electrical activity map by using the body surface signals and the three dimensional cardiac anatomical model and to provide electrocardiographic image data indicative thereof.

The electrocardiographic imaging system is thus advantageously suited to provide a real-time global cardiac electrical activity map in the three-dimensional coordinate space, which requires much less imaging and computational effort to obtain, than in the case of typical electrocardiographic systems that require the use of 60-256 body surface electrodes.

In the electrocardiographic imaging system, the camera unit is configured to generate image data in order to determine the respective position of the three to twenty electrocardiographic electrodes positioned on the body surface of the living being. The electrocardiographic electrodes also provide electrical signals to the electrocardiographic processing unit that are used by the electrocardiographic processing unit to provide the body surface signals indicative of the electrical activity of the heart. The electrocardiographic imaging system is advantageously configured to register information regarding the electrical activity of the heart onto a torso-heart model that is determined by the torso-heart geometry determination system. The torso-heart geometry determination unit uses:
- the determined position of the ultrasound transducer within the three dimensional coordinate space, as determined and provided by transducer position determination system,
- the positions of the electrocardiographic electrodes within the three dimensional coordinate space, as determined and provided by the camera unit, and
- the three dimensional cardiac anatomical model as generated and provided by the ultrasound imaging unit, to determine a position and orientation of the three dimensional shape of the heart within the predetermined three dimensional coordinate space.

The position and orientation of the three-dimensional shape of the heart provided by the torso-heart geometry determination unit is then used by the electrical activity determination unit to generate the electrical activity map. This is done by inverse reconstruction of the electrical activity from the body surface signals onto the three dimensional cardiac anatomical model in the predetermined three-dimensional coordinated space. The electrical activity determination unit then provides electrocardiographic image data that is indicative of the electrical activity map.

In the following, embodiments of the electrocardiographic imaging system of the first aspect of the invention will be described.

In an embodiment, the electrical activity map is generated at the electrical activity determination unit by reconstructing, for instance based on an inverse reconstruction, the body surface signals, indicative of electrical activity of the heart as measured on the surface of the body, onto the three dimensional cardiac anatomical model in the predetermined three dimensional coordinated space

In an embodiment, the camera unit is a 3D camera unit configured to generate image data in the form of 3D photographs, also referred to as three-dimensional images.

In a preferred embodiment, electrical activity determination unit of the electrocardiographic imaging system is configured to generate the electrical activity map in real-time and to provide live electrocardiographic image data indicative thereof.

In a preferred embodiment, the set of electrocardiographic electrodes comprises exactly nine or exactly ten electrocardiographic electrodes and the electrocardiographic processing unit is configured generate and provide a set of twelve body surface signals. This embodiment is particularly suitable for integration into an existing clinical workflow, where a twelve-lead electrocardiographic (12-Lead ECG) system records the heart's electrical activity using exactly nine or exactly ten electrocardiographic electrodes suitably placed according to the existing clinical standards. As of today, the 12-Lead ECG remains a standard diagnostic tool among paramedics, emergency medical technicians, and hospital staff. A 12-Lead ECG provides information pertaining to the heart's electrical activity by acquiring electrical signals through twelve different perspectives or leads using different angles through two electrical planes. The electrical signals are collected by placing the nine or ten electrocardiographic electrodes on the precordial positions of the chest, and on the left and right lateral chest positions and close to the left and right groin positions of the living being. These nine or ten electrocardiographic electrodes are connected to the electrocardiographic processing unit that processes and determines the twelve lead signals.

Thus, this embodiment is particularly suitable for generating a global electrical activity map using an existing twelve-Lead ECG set-up and without requiring changes in the existing clinical work.

In an alternative embodiment, a 5-Lead ECG is used that requires the use of five electrocardiographic electrodes; four limb electrocardiographic electrodes and a chest electrode. The 5-Lead ECG improves ST elevation readings when compared to a 3-Lead ECG used for monitoring a heart rhythm but it is still inferior to the results provided by a 12-Lead ECG.

In an alternative embodiment, exactly 3 electrocardiographic electrodes are used.

In a preferred embodiment of the electrocardiographic imaging system of the first aspect, the ultrasound transducer is configured to obtain the ultrasound image data of the heart from a position on the chest and therefore outside the living being. In this particular embodiment, the transducer position determination unit also comprises the camera unit and is configured to determine the position of the ultrasound transducer from the image data.

Thus, in this embodiment, the camera unit is configured to determine both the respective position of each of the electrocardiographic electrodes and the position of the ultrasound transducer, all of these positions being within the predetermined three-dimensional coordinate space.

This embodiment is suitable for using with any transducer configured to obtain the ultrasound image data from a position outside the living being. For instance, in an embodiment, the ultrasound imaging unit is configured to provide transthoracic echocardiograms (TTE) as ultrasound image data of the heart.

Alternatively, in another embodiment, the ultrasound transducer is configured to obtain the ultrasound image data of the heart from a position inside the living being. The position of the ultrasound transducer cannot be suitably determined by the camera unit. Thus, the transducer position determination unit comprises a tracking system that provides the position of the ultrasound transducer inside the living being. In one embodiment, this can be done by having a reference electromagnetic field generator for generating a reference electromagnetic field and an electromagnetic detector arranged on the ultrasound transducer and configured to detect electromagnetic field gradients.

In another embodiment, the transducer position determination unit consists of a laser and optical fiber, the latter extending into the ultrasound transducer. The three-dimensional position and shape of the optical fiber is determined by means of laser light provided by the laser into the optical fiber and received as reflected light from the optical fiber.

In another embodiment, the transducer position determination unit consists of additional electrode patches on the chest and electrodes on the ultrasound transducer. The three-dimensional position on the electrodes on the transducer can be determined from the voltages generated in those electrodes by electrical fields generated by the chest patches.

Each of the variants of the transducer position determination unit explained above with regard to ultrasound transducer that are placed inside the living being, is also used in a respective embodiment according to the first aspect of the invention to determine the position of an ultrasound transducer positioned on the body surface of the living being.

In a particular embodiment, wherein the ultrasound transducer is configured to obtain the ultrasound image data of the heart from a position inside the living being, the ultrasound imaging unit is configured to provide transesophageal echocardiograms (TEE) or intracardiac echocardiograms (ICE) as ultrasound image data of a heart of a living being.

The electrical activity map provided by any of the embodiments of the first aspect of the present invention can be advantageously used to assist in invasive catheter mapping procedures. An embodiment wherein the electrical activity determination unit is configured to generate the electrical activity map in real-time and to provide live electrocardiographic image data is particularly suitable for assisting in invasive catheter mapping procedures. For instance, cardiac arrhythmias such as ventricular tachycardia (VT), and premature ventricular complexes (PVCs) can be diagnosed and treated by invasive catheter procedures. After introduction of catheters intravascular, in the case of an endocardial approach, or through the thorax, in the case of an epicardial approach, local recordings on the heart surface are usually obtained by contact mapping with the electrodes at the catheter tip. If enough data points are collected, activation isochrones, recovery isochrones, regions of fractionated or low-amplitude electrograms etc. can be visualized on the heart surface, providing localized information for diagnosis and targets for therapy. The catheters contain single or multiple electrodes, and there is a trend to larger numbers of electrodes at the catheter tips to collect more data on multiple locations simultaneously.

However, for every data collection, the catheter needs to be positioned stable during a full beat and will then be moved to a new position. Thus, data collection is point-by-point and beat-by-beat. Especially with the still common single-electrode mapping procedures, collecting data on the relevant region on heart surface can be time consuming, and is not suitable to capture PVCs that occur only seldom or map artificially induced VT's that can be tolerated by a patient for only a very short period.

On the other hand, typical electrocardiographic imaging (ECGI) reconstructs the electrical potentials on the heart surface instantaneous from a number of electrocardiographic electrodes, typically greater than sixty, even up to 256, on the body surface, and a torso-heart geometry that describes the electromagnetic relationship between the body-surface electrocardiographic electrodes and the heart surface. ECGI is noninvasive and instantaneous, and can be used for capturing PVCs and short periods of VT, but its reconstruction of activation isochrones, recovery isochrones and other electrical characteristics is less local and less precise than invasive catheter mapping. Additionally, ECGI requires a change in clinical workflow, as many body-surface electrocardiographic electrodes are required, and a torso-heart geometry needs to be generated.

The electrocardiographic imaging system of the first aspect of the invention enables use of an ECGI implementation with a reduced number of electrocardiographic electrodes and an ultrasound and camera-based method for generating the torso-heart geometry. The electrocardiographic imaging system of the first aspect is suitable for providing an instantaneous global electrical activity map that can be advantageously used for guiding invasive and precise, catheter-based data collection.

This global and instantaneous visualization can aide the operator in determining the regions of interest for the invasive point-by-point data collection with catheters. This potentially reduces the great variability in duration of these procedures, which is partially due to the time-consuming nature of finding the regions where arrhythmias originate. For example, determining the origin of monomorphic PVCs can be cumbersome when such PVCs occur only seldom during the catheter procedure, but could be helped when the global region of origin is shown by the registered electrical activity map generated by the electrical activity determination unit. Additionally, targeting the ablation target of a VT can be hindered by the patient's hemodynamic intolerance to such artificially-induced VT, and may be helped by instantaneous visualization of the electrical activity map of such VT that can be induced, measured and terminated by cardioversion in seconds.

Electrical signals are obtained on the body-surface by a minimal set of electrocardiographic electrodes, preferably the default 12-lead electrocardiographic electrodes as to prevent any changes in the clinical workflow, such as, for instance, the workflow of Vivo, PEACS BV, the Netherlands. The position of these electrocardiographic electrodes is for instance determined by 3D localizing cameras of a 3D camera unit, for example as used in surgical navigation systems, with respect to a common reference. The position and shape of the heart is determined by three-dimensional ultrasound, for instance with either with transthoracic echocardiography (TTE), transesophageal echocardiography (TTE), or intracardiac echocardiography (ICE). The 3D camera unit is used to determine the position of a TTE transducer to the same common spatial reference. However, for TEE or ICE localization methods such as EM tracking or optical shape sensing can be used for the same. By using a common reference for the ultrasound transducer and electrocardiographic electrodes, the 3D heart model obtained with ultrasound imaging unit can be localized relative to the body-surface electrocardiographic electrodes.

The resulting 3D torso-heart geometry then allows reconstructing the electrical potentials on the heart surface instantaneously in the form of an electrical activity map. A real-time visualization of this an electrical activity map may for instance be projected on top of, or next to, the invasive catheter navigation and mapping system in a suitable display device. As such, the real time electrical activity map can guide the invasive data collection.

The electrocardiographic imaging system of the first aspect of the present invention is suitable for assisting in an invasive catheter procedure. For instance, a patient is prepared for the catheter procedure as is common, including attachment of the default ten electrocardiographic electrodes for the twelve lead ECG and the preparation of an invasive three-dimensional mapping and navigation system. A different number of electrocardiographic electrodes can be used but modification of the existing clinical workflow would be required. The camera unit is used to determine the position of the electrocardiographic electrodes on the patient's body surface. The heart shape is determined using an ultrasound imaging unit, for instance with a three dimensional TTE transducer, a three dimensional TEE transducer, or with a three dimensional ICE catheter. When the full geometry model in the three dimensional coordinate space including the position and orientation of the three dimensional cardiac anatomical model is determined by the torso-heart geometry determination unit, the electrical recordings from the body-surface electrocardiographic electrodes will be registered instantaneously and real-time on the cardiac anatomical model, i.e. on the heart geometry, which is then suitable for guiding the collection of more precise invasive data.

According to a third aspect of the present invention, a method for operating an electrocardiographic imaging system is described. The method comprises:
- receiving from an ultrasound transducer ultrasound image data of a heart of a living being;
- generating, based on the ultrasound image data, a three dimensional cardiac anatomical model representing a three dimensional shape of the heart of the living being;
- receiving, from a set of between three and twenty electrocardiographic electrodes positioned on locations on a body surface of the living being, a set of body surface signals indicative of an electrical activity of the heart;
- determining, based on image data generated by a camera unit, a respective position of each of the electrocardiographic electrodes within a predetermined three-dimensional coordinate space;
- determining, using a transducer position determination unit, a position of the ultrasound transducer within the predetermined three dimensional coordinate space;
- determining, based on the determined position of the ultrasound system, the determined positions of the electrocardiographic electrodes and on the three dimensional cardiac anatomical model, a position and orientation of the three dimensional shape of the heart within the predetermined three dimensional coordinate space;
- generating an electrical activity map by inverse reconstruction of the electrical activity of the heart from the body surface signals onto the three dimensional cardiac anatomical model in the predetermined three dimensional coordinated space; and
- providing electrocardiographic image data indicative thereof.

The method of the second aspect shares the advantages of the electrocardiographic imaging system of the first aspect of the invention or of any of its embodiments.

In a preferred embodiment, the method includes receiving the set of body surface signals indicative of an electrical activity of the heart from a set of exactly nine or exactly ten electrocardiographic electrodes positioned on predetermined locations on a body surface of the living being.

According to a forth aspect of the present invention, a computer program is described. The computer program comprises instructions, which, when the program is executed by a computer, cause the computer to carry out the method of the second aspect of the invention.

The computer program of the third aspect of the invention shares the advantages of the electrocardiographic imaging system of the first aspect, of the method of the second aspect or of any of their embodiments.

It shall be understood that the electrocardiographic imaging system of claim 1, the method for operating an electrocardiographic imaging system of claim 8 and the computer program of claim 9, have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

It will be appreciated by those skilled in the art that two or more of the above-mentioned options, implementations, and/or aspects of the invention may be combined in any way deemed useful.

### BRIEF DESCRIPTION OF THE DRAWINGS

In the following drawings:
Fig. 1 shows a schematic block diagram of an embodiment of an electrocardiographic imaging system being used on a patient.
Fig. 2 shows a schematic block diagram of another embodiment of an electrocardiographic imaging system being used on a patient.
Fig. 3 shows a schematic block diagram of another embodiment of an electrocardiographic imaging system being used on a patient.
Fig. 4 shows a flow diagram of an embodiment of a method for controlling operation of an electrocardiographic imaging system.

### DETAILED DESCRIPTION OF EMBODIMENTS

Certain embodiments will now be described in greater details with reference to the accompanying drawings. In the following description, like drawing reference numerals are used for like elements, even in different drawings. The matters defined in the description, such as detailed construction and elements, are provided to assist in a comprehensive understanding of the exemplary embodiments. Also, well-known functions or constructions are not described in detail since they would obscure the embodiments with unnecessary detail. Moreover, expressions such as "at least one of', when preceding a list of elements, modify the entire list of elements and do not modify the individual elements of the list.

The present description generally discloses an electrocardiographic imaging system 100 for providing electrocardiographic image data indicative of an electrical activity map of a heart of a living being 103. Embodiments of an apparatus according to the present invention comprises a plurality of input units for receiving the information enabling an echocardiographic sensing unit (108), a torso-heart geometry determination unit (118) and an electrical activity determination unit (120) as described hereunder. All technical advantages of the system also apply to the apparatus according to the present invention.

The echocardiographic sensing unit (108), the torso-heart geometry determination unit (118) and the electrical activity determination unit (120) may be coupled with each other in different ways, which are not limited to the ones herein described.

The units herein described may be partially collocated in the same hardware component, or may be distributed over multiples hardware components.

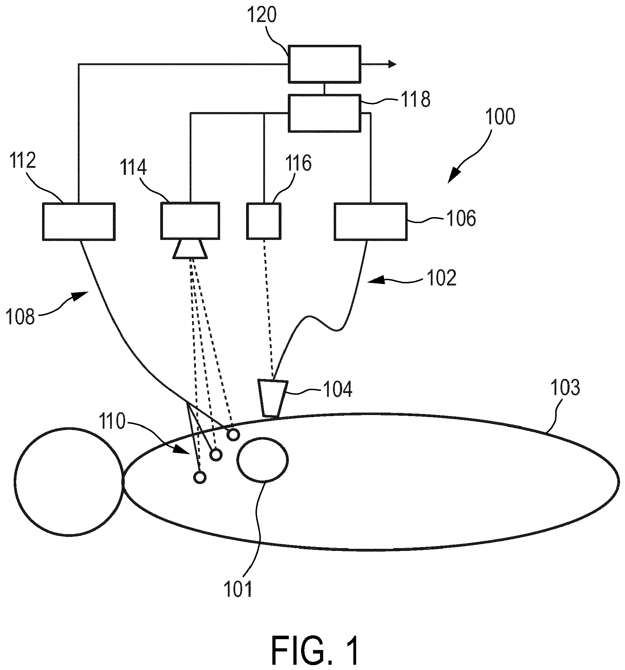

Fig. 1 shows a schematic block diagram of an embodiment of an electrocardiographic imaging system 100 for providing electrocardiographic image data indicative of an electrical activity map of a heart 101 of a living being 103.

The electrocardiographic imaging system 100 comprises an ultrasound imaging unit 102 that includes an ultrasound transducer 104 configured to provide ultrasound image data of the heart 101 of a living being 103 and an ultrasound image data processing 106 unit configured to generate, based on the ultrasound image data, a three dimensional cardiac anatomical model representing a three dimensional shape of the heart of the living being. For instance, the ultrasound imaging unit 106 is configured to provide transthoracic echocardiograms as ultrasound image data of the heart 101 of a living being 103.

The electrocardiographic imaging system 100 also comprises an electrocardiographic sensing 108 unit that includes a set of between three and twenty electrocardiographic electrodes 110 located at predetermined positions on a body surface of the living being for providing electrical signals, and an electrocardiographic processing unit 112.

The electrocardiographic imaging system 100 includes a camera unit 114, which is arranged and configured to generate image data, and to determine, based on the generated image data, a respective position of each of the electrocardiographic electrodes within a predetermined three-dimensional coordinate space. Further, a transducer position determination unit 116 is configured to determine a position of the ultrasound transducer within the predetermined three-dimensional coordinate space.

The determined position of the ultrasound transducer, the determined positions of the electrocardiographic electrodes and the three dimensional cardiac anatomical model are received by a torso-heart geometry determination unit 118 which is configured to determine a position and orientation of the three dimensional shape of the heart within the predetermined three-dimensional coordinate space.

Finally, the electrocardiographic imaging system 100 includes an electrical activity determination unit 120 which is configured to receive, for the torso-heart geometry determination unit, the position and orientation of the three dimensional shape of the heart within the predetermined three dimensional coordinate space and, from the electrocardiographic sensing unit 108, in particular from the electrocardiographic processing unit 112, the body surface electrode signals and to generate the electrical activity map by inverse reconstructing the electrical activity of the heart that is included in the body surface signals onto the three dimensional cardiac anatomical model in the predetermined three dimensional coordinated space. The electrical activity determination unit 120 is also configured to provide electrocardiographic image data indicative thereof.

Preferably, the set of electrocardiographic electrodes 110 comprises exactly nine or exactly ten electrocardiographic electrodes and the electrocardiographic processing unit 112 is configured generate and provide a set of twelve lead signals in according with common ECG practice and related clinical workflow.

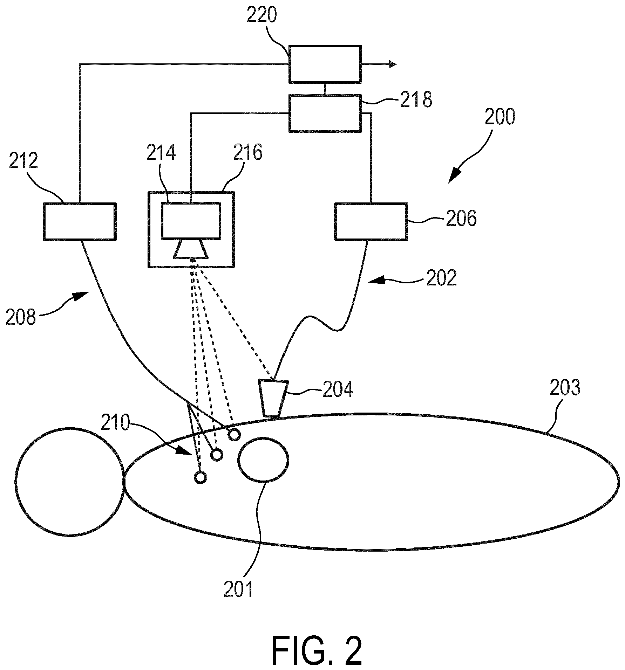

Fig. 2 shows a schematic block diagram of another embodiment of an electrocardiographic imaging system 200 for providing electrocardiographic image data indicative of an electrical activity map of a heart 201 of a living being 203. The following discussion is focused on the features distinguishing the electrocardiographic imaging system 200 of Fig. 2 from electrocardiographic imaging system 100 of Fig. 1. Features common to both the electrocardiographic imaging system 100 and electrocardiographic imaging system 200 are referred to using the same numerals except for the first digit, which is "1" for electrocardiographic imaging system 100 of Fig. 1 and "2" for the electrocardiographic imaging system 200 of Fig.2

In the exemplary electrocardiographic imaging system 200, the ultrasound transducer 204 is configured to obtain the ultrasound image data of the heart 201 from a position outside the living being 203. Further, the transducer position determination unit 216 comprises the camera unit 214 and is configured to determine the position of the ultrasound transducer from the image data. Thus, the camera unit is advantageously configured to determine the position of both the set of electrocardiographic electrodes and of the ultrasound transducer and to provide information pertaining to said positions to the torso-heart geometry determination unit 218.

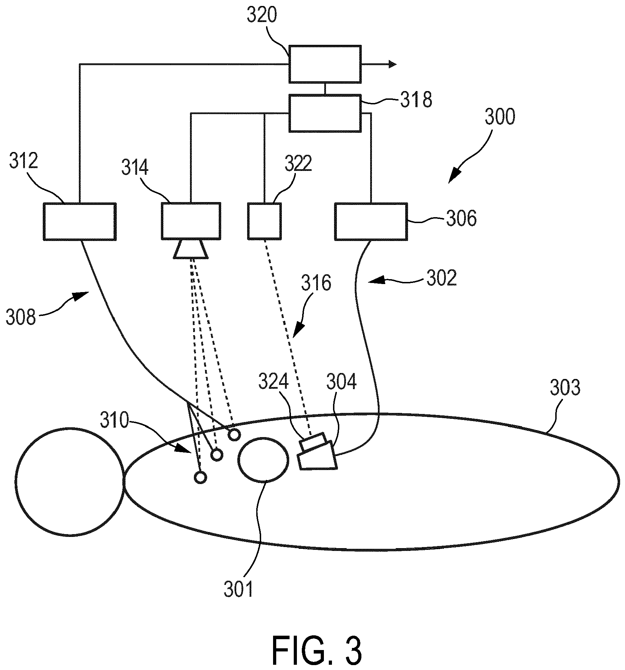

Fig. 3 shows a schematic block diagram of yet another embodiment of an electrocardiographic imaging system 300 for providing electrocardiographic image data indicative of an electrical activity map of a heart 301 of a living being 303. The following discussion is focused on the features distinguishing the electrocardiographic imaging system 300 of Fig. 3 from electrocardiographic imaging systems 100 and 200 of Figs. 1 and 2 respectively. Features common to electrocardiographic imaging systems 100, 200 and 300 are referred to using the same numerals except for the first digit, which is "1" and "2" for electrocardiographic imaging systems 100 and 200 of Figs. 1 and 2 and "3" for the electrocardiographic imaging system 300 of Fig.3

In the electrocardiographic imaging system 300, the ultrasound transducer 304 is configured to obtain the ultrasound image data of the heart 301 of the living being 303 from a position inside the living being. For instance, the ultrasound imaging unit 302 is configured to provide transesophageal echocardiograms (TEE) or intracardiac echocardiograms (ICE) as ultrasound image data of the heart of the living being. A position of the ultrasound transducer inside the living being is determined and tracked the transducer position determination unit 316 that comprises an electromagnetic tracking system having a reference electromagnetic field generator 322 for generating a reference electromagnetic field and an electromagnetic detector 324 arranged on the ultrasound transducer and configured to detect electromagnetic field gradients.

In another electrocardiographic imaging system (not shown), the transducer position determination unit consists of a laser and optical fiber, the latter extending into the ultrasound transducer. The three-dimensional position and shape of the optical fiber is determined by means of laser light provided by the laser into the optical fiber and received as reflected light from the optical fiber.

In yet another electrocardiographic imaging system (not shown), the transducer position determination unit consists of additional electrode patches on the chest and electrodes on the ultrasound transducer. The three-dimensional position on the electrodes on the transducer can be determined from the voltages generated in those electrodes by electrical fields generated by the chest patches.

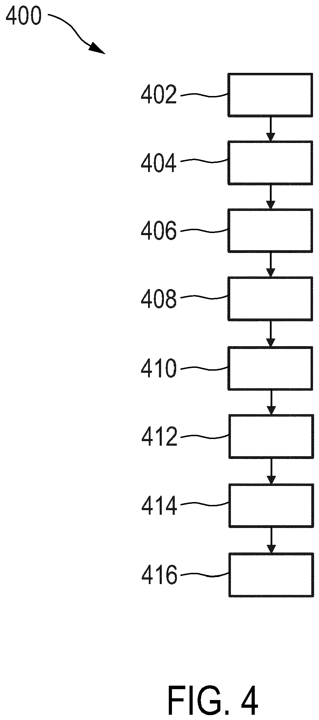

Fig. 4 shows a flow diagram of an embodiment of a method 400 for controlling operation of an electrocardiographic imaging system. The method comprises receiving, in a step 402, from an ultrasound transducer ultrasound, image data of a heart of a living being. The method also comprises generating, in a step 404, and based on the ultrasound image data, a three dimensional cardiac anatomical model representing a three dimensional shape of the heart of the living being. The method also comprises receiving, in a step 406, and from a set of between three and twenty electrocardiographic electrodes, preferably exactly nine or ten electrocardiographic electrodes, a set of body surface signals indicative of an electrical activity of the heart. The method also comprises determining, in a step 408, and based on image data generated by a camera unit, a respective position of each of the electrocardiographic electrodes within a predetermined three-dimensional coordinate space. Further, the method comprises determining, in a step 410, and using a transducer position determination unit, a position of the ultrasound transducer within the predetermined three dimensional coordinate space. The order in which the steps 404, 406, 408 and 410 are carried out is not determining.

The method then includes a step 412 in which, based on the determined position of the ultrasound system, the determined positions of the electrocardiographic electrodes and on the three dimensional cardiac anatomical model, a position and orientation of the three-dimensional shape of the heart within the predetermined three dimensional coordinate space is determined. The method also comprises generating, in a step 414 an electrical activity map by inverse reconstruction of the electrical activity of the heart from the body surface signals onto the three dimensional cardiac anatomical model in the predetermined three dimensional coordinated space; and, in a step 416, providing electrocardiographic image data indicative thereof. The heart of a living being is an electrically active organ and generates electrical potentials. These are propagated through the body to the body surface of the human being, where they are recorded by the set of electrocardiographic electrodes positioned on respective predetermined locations. It is per se known to model how these electrical potentials propagate from the heart to the body. The model is then inverted to reconstruct the electrical activity of the heart by using the set of body surface signals recorded on the body surface and provided by electrocardiographic processing unit. This is herein referred to as the "inverse problem" of electrocardiography and allows an electrocardiographic imaging (ECGI).

Aspects of the invention may be implemented in a computer program product, which may be a collection of computer program instructions stored on a computer readable storage device, which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention maybe distributed over multiple computers or processors.

As discussed above, the processing unit, for instance a controller implements the control method. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform at the required functions. Various storage media may be fixed within a processor or controller or may be transportable, such that the one or more programs stored thereon can be loaded into a processor or controller. While the invention has been illustrated and described in detail in the drawings and foregoing description, such illustration and description are to be considered illustrative or exemplary and not restrictive; the invention is not limited to the disclosed embodiments.

In summary the invention relates to an electrocardiographic imaging system for providing electrocardiographic image data indicative of an electrical activity map of a heart suitable for real time applications, and that comprises an ultrasound imaging unit for generating a three dimensional cardiac anatomical model, an electrocardiographic sensing unit comprising a set of between three and twenty electrocardiographic electrodes for providing body surface signals indicative of an electrical activity of the heart, a camera unit for determining positions of the electrocardiographic electrodes , a transducer position determination system, for determining a position of the ultrasound transducer, a torso-heart geometry determination unit for determining a position and orientation of the three dimensional shape of the heart within a predetermined three dimensional coordinate space and an electrical activity determination unit for generating an electrical activity map of the heart, suitable for real time applications.

In the claims, the word "comprising" does not exclude other elements or steps, and the indefinite article "a" or "an" does not exclude a plurality.

A single step or other units may fulfill the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. An electrocardiographic imaging apparatus, comprising:
- an ultrasound input unit for receiving a three dimensional cardiac anatomical model representing a three dimensional shape of the heart of the living being based on ultrasound image data;
- a camera input unit for receiving a respective position of each of the electrocardiographic electrodes within a predetermined three dimensional coordinate space based on image data;
- a transducer input unit for receiving a position of the ultrasound transducer within the predetermined three dimensional coordinate space
- an echocardiographic sensing unit (108) comprising
- a set of between three and twenty electrocardiographic electrodes (110) for providing electrical signals; and
- an electrocardiographic processing unit (112) configured to provide, when the set of electrocardiographic electrodes is positioned on a body surface of the living being, a set of body surface signals indicative of an electrical activity of the heart;
- a torso-heart geometry determination unit (118) which is configured
- to determine, based on (i) the determined position of the ultrasound transducer, (ii) the determined positions of the electrocardiographic electrodes and (iii) the three dimensional cardiac anatomical model, a position and orientation of the three dimensional shape of the heart within the predetermined three dimensional coordinate space; and
- an electrical activity determination unit (120) which is configured:
- to receive (i) the position and orientation of the three dimensional shape of the heart within the predetermined three dimensional coordinate space and (ii) the set of body surface signals;
- to generate an electrical activity map by using the body surface signals and the three dimensional cardiac anatomical model; and
- provide electrocardiographic image data indicative thereof.

2. An electrocardiographic imaging system (100), comprising:
- an ultrasound imaging unit (102) which comprises:
- an ultrasound transducer (104) configured to provide ultrasound image data of a heart (101) of a living being (103); and
- an ultrasound image data processing unit (106) configured to generate, based on the ultrasound image data, a three dimensional cardiac anatomical model representing a three dimensional shape of the heart of the living being;
- an electrocardiographic sensing unit (108) comprising:
- a set of between three and twenty electrocardiographic electrodes (110) for providing electrical signals; and
- an electrocardiographic processing unit (112) configured to provide, when the set of electrocardiographic electrodes is positioned on a body surface of the living being, a set of body surface signals indicative of an electrical activity of the heart;
- a camera unit (114) which is configured to generate image data and to determine, based on the generated image data, a respective position of each of the electrocardiographic electrodes within a predetermined three dimensional coordinate space;
- a transducer position determination unit (116), which is configured to determine a position of the ultrasound transducer within the predetermined three dimensional coordinate space;
- a torso-heart geometry determination unit (118) which is configured
- to determine, based on (i) the determined position of the ultrasound transducer, (ii) the determined positions of the electrocardiographic electrodes and (iii) the three dimensional cardiac anatomical model, a position and orientation of the three dimensional shape of the heart within the predetermined three dimensional coordinate space; and
- an electrical activity determination unit (120) which is configured:
- to receive (i) the position and orientation of the three dimensional shape of the heart within the predetermined three dimensional coordinate space and (ii) the set of body surface signals;
- to generate an electrical activity map by using the body surface signals and the three dimensional cardiac anatomical model; and
- to provide electrocardiographic image data indicative thereof.

3. The electrocardiographic imaging system of claim 2, wherein the set of electrocardiographic electrodes comprises exactly nine or exactly ten electrocardiographic electrodes and the electrocardiographic processing unit is configured generate and provide a set of twelve body surface signals.

4. The electrocardiographic imaging system of claim 2,
- wherein the ultrasound transducer (204) is configured to obtain the ultrasound image data of the heart from a position outside the living being; and
- wherein the transducer position determination unit (216) comprises the camera unit (214) and is configured to determine the position of the ultrasound transducer from the image data;

5. The electrocardiographic imaging system of claim 4, wherein the ultrasound imaging unit is configured to provide transthoracic echocardiograms as ultrasound image data of the heart of the living being.

6. The electrocardiographic imaging system of claim 2,
- wherein the ultrasound transducer is configured to obtain the ultrasound image data of the heart from a position inside the living being; and
- wherein the transducer position determination unit comprises an electromagnetic tracking system having a reference electromagnetic field generator (322) for generating a reference electromagnetic field and an electromagnetic detector (324) arranged on the ultrasound transducer and configured to detect electromagnetic field gradients.

7. The electrocardiographic imaging system of claim 6, wherein the ultrasound imaging unit is configured to provide transesophageal echocardiograms or intracardiac echocardiograms as ultrasound image data of the heart of the living being.

8. The electrocardiographic imaging system of claim 2, wherein the electrical activity determination unit (120) is configured to generate the electrical activity map in real-time and to provide live electrocardiographic image data indicative thereof.

9. A method (400) for operating an electrocardiographic imaging system, the method comprising:
- receiving (402) from an ultrasound transducer ultrasound image data of a heart of a living being;
- generating (404), based on the ultrasound image data, a three dimensional cardiac anatomical model representing a three dimensional shape of the heart of the living being;
- receiving (406), from a set of between three and twenty electrocardiographic electrodes positioned on a body surface of the living being, a set of body surface signals indicative of an electrical activity of the heart;
- determining (408), based on image data generated by a camera unit, a respective position of each of the electrocardiographic electrodes within a predetermined three dimensional coordinate space;
- determining (410), using a transducer position determination unit, a position of the ultrasound transducer within the predetermined three dimensional coordinate space;
- determining (412), based on (i) the determined position of the ultrasound system, (ii) the determined positions of the electrocardiographic electrodes and (iii) the three dimensional cardiac anatomical model, a position and orientation of the three dimensional shape of the heart within the predetermined three dimensional coordinate space;
- generating (414) an electrical activity map by using the body surface signals and the three dimensional cardiac anatomical; and
- providing (416) electrocardiographic image data indicative thereof.

10. A computer program comprising instructions which, when the program is executed by a computer, cause the computer to carry out the method of claim 9.
